# EUROPÄISCHE PATENTSCHRIFT

(11) **EP 0 705 615 B1**
(45) Veröffentlichungstag und Bekanntmachung des Hinweises auf die Patenterteilung: **02.07.2003**
(21) Anmeldenummer: 95113816.3
(22) Anmeldetag: 02.09.1995
(51) Int. Cl.: A61M 16/00, A61B 5/085

(54) **Beatmungsgerät zur Therapie der Schlafapnoe**
Ventilation apparatus for the treatment of sleep apnoea
Appareil de ventilation artificielle pour le traitement de l'apnée du sommeil

(30) Priorität: 08.09.1994 DE 9414568 U
(43) Veröffentlichungstag der Anmeldung: 10.04.1996
(60) Teilanmeldung: 03001806.3
(73) Patentinhaber: GOTTLIEB WEINMANN GERÄTE FÜR MEDIZIN UND ARBEITSSCHUTZ GMBH & CO., 22525 Hamburg (DE)
(72) Erfinder: Graetz, Bernd, D-22869 Schenefeld (DE); Maurer, Jörg, D-22113 Oststeinbek (DE)
(74) Vertreter: Hansmann, Dierk, Dipl.-Ing.

(56) Entgegenhaltungen:
- EP-A- 0 373 585
- US-A- 4 031 885
- US-A- 5 245 995
- US-A- 5 318 038

## Beschreibung

Die Erfindung betrifft ein Beatmungsgerät zur Atmungsunterstützung bei einem Auftreten von Symptomen der Schlafapnoe, bestehend aus einer mit einer Atemmaske verbundenen Druckgasquelle.

Eine nicht unbeachtliche Anzahl von Menschen leiden unter Schlafstörungen, die sich auf das Tagesbefinden dieser Menschen auswirken und deren soziale und berufliche Leistungsfähigkeit sowie deren Lebensqualität zum Teil erheblich beeinträchtigen. Eine dieser Schlafstörungen ist die Schlafapnoe, die vorrangig mit der sogenannten CPAP-Therapie (CPAP = Continuous Positive Airway Pressure) behandelt wird, indem dem Patienten während des Schlafes ein Luftstrom aus einem atemfähigen Gas über eine Nasalmaske kontinuierlich zugeführt wird. Die Maske ist über einen Schlauch mit einem Beatmungsgerät verbunden, das einen Lüfter umfaßt, der einen Gasstrom mit einem Überdruck von 5 bis 20 mbar erzeugt.

Der Gasstrom wird dem Patienten entweder mit konstantem Druck zugeführt, oder zur Erleichterung der Atemarbeit des Patienten beim Ausatmen auf ein niedrigeres Druckniveau abgesenkt. Obwohl Schlafapnoen nur kurzfristig auftreten und einen geringen Teil des Schlafes ausmachen, läuft der Lüfter bei beiden Verfahren während der gesamten Schlafdauer (Nacht), was die Akzeptanz dieser Schlafapnoe-Behandlung erschwert.

Aus der US-A-5 245 995 ist ein CPAP-Beatmungsgerät bekannt, das für Patienten mit Schlafapnoe einsetzbar ist. Das Atemgas wird dem Patienten über eine Atemmaske zugeführt und im Bereich des Gerätes ist eine Druckgasqueile angeordnet. Die Druckgasquelle ist in Abhängigkeit vom Atemwiderstand steuerbar.

Aus der EP-A-0 373 585 ist es bekannt, den Atemwiderstand eines Patienten mit Hilfe der ORM-Messung durchzuführen. Mit vorgebbarer Frequenz wird hierbei dem Atemvolumenstrom ein oszillierender Volumenstrom mit geringem Volumenhub überlagert. Aus der mit gleicher Frequenz auftretenden periodischen Druckschwankung kann ein vom konkreten Atemwiderstand abhängiger Meßwert bereitgestellt werden.

Aus der US-A-5 318 038 ist es bekannt, eine Atemmessung unter Verwendung eines Pneumotachographen in der Gaszuführleitung durchzuführen.

Aufgabe der Erfindung ist es nun, ein Beatmungsgerät für die CPAP-Therapie patientenfreundlich zu gestalten.

Mit dem gerät kann das folgende Verfahren durch geführt werden, gemäß dem mit der Oszilloresistometrie (oszillatorische Resistenz-Messung = ORM) die oszillatorische Druckamplitude, die dem Atemwiderstand des Patienten entspricht, kontinuierlich gemessen wird, wobei nach dem Bestimmen des individuellen Atemwiderstandswertes (Basiswert der Druckamplitude) bei Abweichungen von diesem Wert dem Patienten Atemgas unter Druck zugeführt und die Gaszufuhr beendet wird, sobald der Basiswert wieder oder annähernd erreicht ist.

Diese Aufgabe wird erfindungsgemäß durch ein Beatmungsgerät nach Anspruch 1 gelöst.

Bei dem Verfahren zur Steuerung und Regelung eines Beatmungsgerätes zur Atmungsunterstützung von Schlafapnoe-Patienten wird dieses Gerät nur dann aktiviert, d.h. es wird dem Patienten nur dann Atemgas zugeführt, wenn durch eine Apnoe die Atemtätigkeit des Patienten gestört ist oder sich der Atemwiderstand - und somit auch die gemessene oszillatorische Druckamplitude-durch eine entstehende Apnoe ändert. Eine Störung der Atemtätigkeit des Patienten ist von einer Veränderung des Atemwiderstandes des Patienten begleitet. Dieser Widerstand wird ohne das Befinden des Patienten zu beeinflussen, auf einfache Weise zuverlässig und reproduzierbar über die Veränderungen der oszillatorischen Druckamplitude bestimmt. Diese bildet die Steuergröße für das Ein- und Ausschalten des Behandlungsgerätes, so daß eine Unterversorgung des Patienten mit Sauerstoff nicht eintritt.

Das Befinden des Patienten kann bei einer vorteilhaften Weiterbildung des Beatmungsgerätes noch dadurch gesteigert werden, daß dem Patienten ständig Atemgas mit einem mäßigen Grunddruck von 3 bis 5 mbar zugeführt, der Gasdruck bei Abweichungen vom individuellen Atemwiderstandswert als Basiswert angehoben und wieder auf den Grunddruck abgesenkt wird, sobald der Basiswert wieder annähernd erreicht ist. Diese kontinuierliche Gaszufuhr unterstützt nicht nur die Atemarbeit des Patienten wohltuend, sondern wirkt sich sogleich vorteilhaft auf die Ermittlung der zur Steuerung des Druckes des Atemgases erforderlichen Parameter aus.

Im Verlauf einer beginnenden Apnoe bewirken Verengungen oder Erweiterungen der Atemwege eines Patienten Änderungen im Phasenwinkel der oszillatorischen Druckamplitude und des Atemflusses im Vergleich zu den entsprechenden Basiswerten bei normalem oder ungestörtem Atemhub. Diesen unerwünschten Veränderungen kann mit weiteren zweckmäßigen Ausgestaltungen der Erfindung dadurch begegnet werden, daß als zusätzliche Steuer- und/oder Regelsignale für das Beatmungsgerät der Phasenwinkel der Druckamplitude und/oder der Atemfluß des Patienten herangezogen werden, um bei signifikanten Abweichungen von den jeweiligen - zu Beginn der Therapie ermittelten - individuellen Basiswerten den Druck des zugeführten Atemgases so zu erhöhen, bis die Basiswerte wieder oder annähernd erreicht sind.

Ein Beatmungsgerät besteht aus einer mit einer Atemmaske verbundenen und zweckmäßigerweise als Lüfter ausgebildeten Druckgasquelle und ist durch eine Einrichtung zur kontinuierlichen Messung der oszillatorischen Druckamplitude eines Patienten nach dem ORM-Prinzip sowie durch eine Steuer-Regeleinrichtung gekennzeichnet, die auf den individuellen Basiswert der dem Atemwiderstandswert eines Patienten entsprechenden oszillatorischen Druckamplitude einstellbar ist und die Druckgasquelle so aktiviert, daß dem Patienten Atemgas zugeführt wird, wenn signifikante Abweichungen des Atemwiderstandes vom Basiswert auftreten.

Bei einer zweckmäßigen Ausgestaltung kann das Beatmungsgerät als weitere Steuerelemente zum Aktivieren der Druckgasquelle noch eine Einrichtung zum Messen und Bestimmen des Phasenwinkels der Druckamplitude und/oder eine als Pneumotachograph oder Meßblende ausgebildete Einrichtung zum Bestimmen des Atemflusses des Patienten aufweisen.

Bei einer anderen Weiterbildung der Erfindung kann das Beatmungsgerät noch mit einem auf die Bedürfnisse des Patienten einstellbaren Druckregler für das Atemgas versehen sein, wodurch die Akzeptanz des Therapiegerätes gesteigert wird, da der Patient mit einem für ihn angenehmen konstanten Druck beatmet wird, wenn die oszillatorische Druckamplitude und/oder der Phasenwinkel der Druckamplitude und/oder der Atemfluß des Patienten dem (den) Basiswert(en) entspricht (entsprechen).

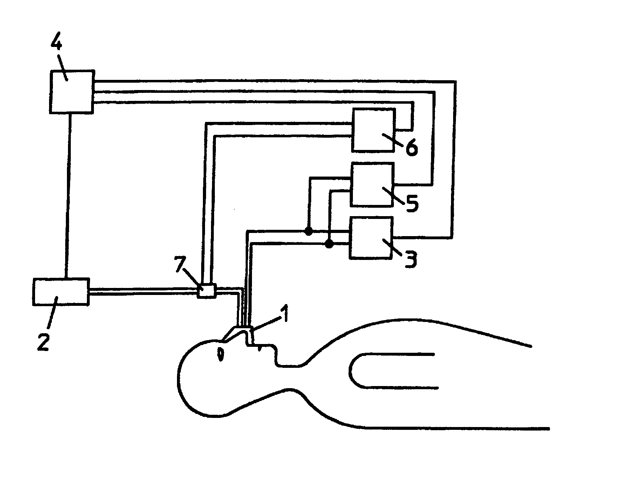

Ein Ausführungsbeispiel des erfindungsgemäßen Beatmungsgerätes wird noch an Hand der Zeichnung, in der es schematisch dargestellt ist, beschrieben.

Das Beatmungsgerät zur Therapie der Schlafapnoe weist eine auf der Nase eines Patienten anordbare Atemmaske (1) auf, die über einen Atemschlauch (8) mit einer als Lüfter ausgebildeten Druckgasquelle (2) verbunden ist. In der Atemmaske (1) sind die Sensoren (nicht gezeigt) einer Einrichtung (3) zum kontinuierlichen Messen der oszillatorischen Druckamplitude nach dem ORM-Prinzip vorgesehen. Von einer Steuer-Regeleinrichtung (4), die mit der Einrichtung (3) verbunden und auf den individuellen Basiswert der dem Atemwiderstand des Patienten entsprechenden oszillatorischen Druckamplitude einstellbar ist, wird die Druckgasquelle (2) so aktiviert, daß dem Patienten Atemgas bei signifikanten Abweichungen der Druckamplitude vom Basiswert zugeführt wird. In den Atemschlauch (8) ist eine Meßblende (7) eingesetzt, die den Atemfluß erfaßt und die entsprechenden Daten über eine Meßeinrichtung (6) an die Steuer-Regeleinrichtung (4) zur weiteren Beeinflussung der Druckgasquelle (2) weiterleitet. In der Atemmaske (1) sind außerdem noch die Sensoren (nicht dargestellt) einer anderen Einrichtung (5) zum Messen des Phasenwinkeis der Druckamplitude vorgesehen. Die Einrichtung (5) sendet an die Steuer-Regeleinrichtung (4) Signale, die als zusätzliche Regelparameter bei der Aktivierung der Druckquelle (2) dienen.

## Patentansprüche

1. Beatmungsgerät zur Atmungsunterstützung bei einem Auftreten von Symptomen der Schlafapnoe, bestehend aus einer mit einer Atemmaske verbundenen Druckgasquelle, **dadurch gekennzeichnet, daß** eine Einrichtung (3) zur kontinuierlichen Messung der von einem Atemwiderstand abhängigen oszillatorischen Druckamplitude nach dem ORM-Prinzip vorgesehen ist, die mit einer Steuer-Regeleinrichtung (4) verbunden ist die auf den individuellen Basiswert der dem Atemwiderstands wert eines Patienten entspsechenden oszillatorischen Druchamplitude einstellbar ist und bei der die Steuer-Regeleinrichtung (4), die Druckgasquelle (2) derart aktiviert bzw. steuert, daß der Atemmaske bei vorgebbaren signifikanten Abweichungen der oszillatorischen Druckamplitude vom Basiswert Atemgas mit erhöhtem Druck zugeführt wird.

2. Beatmungsgerät nach Anspruch 1, **dadurch gekennzeichnet, daß** die Druckgasquelle (2) ein Lüfter ist.

3. Beatmungsgerät nach Anspruch 1 oder 2, **gekennzeichnet durch** einen einstellbaren Druckregler für das Atemgas.

4. Beatmungsgerät nach einem der Ansprüche 1 bis 3, **gekennzeichnet durch** eine Einrichtung (5) zur kontinuierlichen Messung und Bestimmung des Phasenwinkels der Druckamplitude, welche Einrichtung (5) mit der Steuer-Regeleinrichtung (4) verbunden ist, die auf den individuellen Basiswert des Phasenwinkels einstellbare Elemente aufweist.

5. Beatmungsgerät nach einem der Ansprüche 1 bis 4, **gekennzeichnet durch** eine mit einer Meßblende (7) oder einem Pneumotachographen in der Gaszuführleitung (8) verbundene Meßeinrichtung (6) zur kontinuierlichen Messung und Bestimmung des Atemflusses **durch** die Atemmaske hindurch, wobei die Meßeinrichtung (6) mit der Steuer-Regeleinrichtung (4) verbunden ist, die auf den individuellen Basiswert des Atemflusses einstellbare Elemente aufweist.

6. Beatmungsgerät nach Anspruch 4 oder 5, **dadurch gekennzeichnet, daß** in der Einrichtung (5) zur kontinuierlichen Messung und Bestimmung des Phasenwinkels aus Signalen von der Einrichtung (3) zur kontinuierlichen Messung der oszillatorischen Druckamplitude und Signalen der Meßblende (7) oder des Pneumotachographen der Phasenwinkel zwischen dem oszillatorischen Volumenstrom und dem oszillatorischen Druck bestimmt wird.

## Claims

1. Respiratory appliance for breathing assistance on presentation of the symptoms of sleep apnoea, comprising a compressed-gas source connected to a breathing mask, **characterized in that** a device (3) for continuous measurement of the oscillatory pressure amplitude as a function of a resistance to respiration according to the ORM principle is provided, which is connected to a control/regulating device (4), which is adjustable to the individual basic value of the oscillatory pressure amplitude corresponding to the respiration resistance value of a patient and in which the control/regulating device (4) activates and/or controls the compressed-gas source (2) in such a way that in the event of preselectable significant variations of the oscillatory pressure amplitude from the basic value respiratory gas is supplied at an increased pressure to the breathing mask.

2. Respiratory appliance according to claim 1, **characterized in that** the compressed-gas source (2) is a fan.

3. Respiratory appliance according to claim 1 or 2, **characterized by** an adjustable pressure regulator for the respiratory gas.

4. Respiratory appliance according to one of claims 1 to 3, **characterized by** a device (5) for continuous measurement and determination of the phase angle of the pressure amplitude, which device (5) is connected to the control/regulating device (4), which comprises elements adjustable to the individual basic value of the phase angle.

5. Respiratory appliance according to one of claims 1 to 4, **characterized by** a measuring device (6), which is connected to a measuring orifice (7) or a pneumotachograph in the gas supply line (8), for continuous measurement and determination of the respiratory flow through the breathing mask, wherein the measuring device (6) is connected to the control/regulating device (4), which comprises elements adjustable to the individual basic value of the respiratory flow.

6. Respiratory appliance according to claim 4 or 5, **characterized in that** in the device (5) for continuous measurement and determination of the phase angle the phase angle between the oscillatory volume rate of flow and the oscillatory pressure is determined from signals from the device (3) for continuous measurement of the oscillatory pressure amplitude and from signals of the measuring orifice (7) or pneumotachograph.

## Revendications

1. Appareil de ventilation pour la respiration artificielle pour le traitement des symptômes de l'apnée du sommeil, consistant en une source de gaz comprimé reliée à un masque, **caractérisé en ce qu'**un dispositif (3) pour la mesure continue de l'amplitude oscillante de pression, dépendant d'une résistance respiratoire selon le principe ORM, est prévu, lequel est relié à un dispositif de réglage (4), qui peut être réglé sur la valeur de base individuelle de résistance respiratoire d'un patient correspondant à l'amplitude oscillante de pression, et pour lequel le dispositif de réglage (4) active ou règle la source de gaz comprimé (2) de sorte que le masque soit alimenté en gaz respirable sous haute pression lors de divergences significatives prédéterminables de l'amplitude oscillante de pression.

2. Appareil de ventilation selon la revendication 1, **caractérisé en ce que** la source de gaz comprimé (2) est un aérateur.

3. Appareil de ventilation selon la revendication 1 ou 2, **caractérisé par** un régulateur de pression ajustable pour le gaz respirable.

4. Appareil de ventilation selon l'une quelconque des revendications 1 à 3, **caractérisé par** un dispositif (5) pour la mesure continue et la détermination de l'angle de phase de l'amplitude de pression, lequel dispositif (5) est relié au dispositif de réglage (4), qui présente des éléments ajustables à la valeur de base individuelle de l'angle de phase.

5. Appareil de ventilation selon l'une quelconque des revendications 1 à 4, **caractérisé par** un dispositif de mesure (6), relié à la conduite d'alimentation en gaz (8) par un diaphragme de mesure (7) ou un pneumotachographe, pour la mesure continue et la détermination du flux de respiration traversant le masque, où le dispositif de mesure (6) est relié au dispositif de réglage (4), qui présente des éléments ajustables à la valeur de base individuelle du flux de respiration.

6. Appareil de ventilation selon la revendication 4 ou 5, **caractérisé en ce que** dans le dispositif (5) pour la mesure continue et la détermination de l'angle de phase, on détermine l'angle de phase entre le courant volumique oscillant et la pression oscillante à partir des signaux du dispositif (3) pour la mesure continue de l'amplitude oscillante de pression et des signaux du diaphragme de mesure (7) ou du pneumotachographe.
